# EUROPEAN PATENT SPECIFICATION

(11) **EP 2 473 599 B1**
(45) Date of publication and mention of the grant of the patent: **17.10.2018**
(21) Application number: 10749856.0
(22) Date of filing: 02.09.2010
(51) Int. Cl.: A61K 39/12, C07K 14/005, C12N 7/00, A61K 39/00, A61K 39/245

(54) **BOVINE HERPESVIRUS VACCINE**
KUHHERPESVIRUSIMPFSTOFF
VACCIN CONTRE L'HERPÈS-VIRUS BOVIN

(30) Priority: 03.09.2009 EP 09169358; 04.09.2009 US 239894 P
(43) Date of publication of application: 11.07.2012
(73) Proprietor: Intervet International B.V., 5831 AN Boxmeer (NL)
(72) Inventor: VANDERPLASSCHEN, Alain, Francis, Claude, B-4000 Liège (BE); THIRION, Muriel, Michèle, Chantal, B-4000 Liège (BE)
(74) Representative: Intervet International B.V.
(86) International application number: PCT/EP2010/062846
(87) International publication number: WO 2011/026882

(56) References cited:
- GILLET L ET AL: "Development of bovine herpesvirus 4 as an expression vector using bacterial artificial chromosome cloning" JOURNAL OF GENERAL VIROLOGY, vol. 86, April 2005 (2005-04), pages 907-917, XP002593831 ISSN: 0022-1317
- LOMONTE P ET AL: "ANALYSIS OF BOVINE HERPESVIRUS 4 GENOMIC REGIONS LOCATED OUTSIDE THE CONSERVED GAMMAHERPESVIRUS GENE BLOCKS" JOURNAL OF GENERAL VIROLOGY, SOCIETY FOR GENERAL MICROBIOLOGY, SPENCERS WOOD, GB, vol. 76, 1 January 1995 (1995-01-01), pages 1835-1841, XP001053327 ISSN: 0022-1317
- DEWALS BENJAMIN ET AL: "Evolution of Bovine herpesvirus 4: recombination and transmission between African buffalo and cattle" JOURNAL OF GENERAL VIROLOGY, vol. 87, no. Part 6, June 2006 (2006-06), pages 1509-1519, XP002593832 ISSN: 0022-1317
- THIRION M ET AL: "Bovine herpesvirus 4 ORF73 is dispensable for virus growth in vitro, but is essential for virus persistence in vivo." THE JOURNAL OF GENERAL VIROLOGY OCT 2010 LNKD- PUBMED:20592111, vol. 91, no. Pt 10, 30 June 2010 (2010-06-30), pages 2574-2584, XP002603988 ISSN: 1465-2099
- A. Grundhoff ET AL: "The Latency-Associated Nuclear Antigen of Kaposi's Sarcoma-Associated Herpesvirus Permits Replication of Terminal Repeat-Containing Plasmids", JOURNAL OF VIROLOGY., vol. 77, no. 4, 15 February 2003 (2003-02-15), pages 2779-2783, XP055280118, US ISSN: 0022-538X, DOI: 10.1128/JVI.77.4.2779-2783.2003

### SEQUENCE LISTING

<110> Intervet International B.V.
<120> Bovine herpesvirus vaccine
<130> 2009.026
<160> 27
<170> PatentIn version 3.5
<210> 1
   <211> 22
   <212> DNA
   <213> Bovine herpesvirus 4
<400> 1
   tcatgtcctg agtgggtcta tg 22
<210> 2
   <211> 23
   <212> DNA
   <213> Bovine herpesvirus 4
<400> 2
   gctacagaaa atggccagta aag 23
<210> 3
   <211> 20
   <212> DNA
   <213> Bovine herpesvirus 4
<400> 3
   catggagagg ccatcaggag 20
<210> 4
   <211> 22
   <212> DNA
   <213> Bovine herpesvirus 4
<400> 4
   cttaaccaca gaactccttt gc 22
<210> 5
   <211> 20
   <212> DNA
   <213> Bovine herpesvirus 4
<400> 5
   ccgggtgaaa caagttcctg 20
<210> 6
   <211> 24
   <212> DNA
   <213> Bovine herpesvirus 4
<400> 6
   ggtcagagaa catatgataa catc 24
<210> 7
   <211> 29
   <212> DNA
   <213> Bovine herpesvirus 4
<400> 7
   ccaagcttct agagagtgat ttgtagtgt 29
<210> 8
   <211> 30
   <212> DNA
   <213> Bovine herpesvirus 4
<400> 8
   aaggatccga tggttaccag ggatgtgttg 30
<210> 9
   <211> 21
   <212> DNA
   <213> Bovine herpesvirus 4
<400> 9
   tcactgatca acgggtattc c 21
<210> 10
   <211> 18
   <212> DNA
   <213> Bovine herpesvirus 4
<400> 10
   atgccgggcc gccacctc 18
<210> 11
   <211> 19
   <212> DNA
   <213> Bovine herpesvirus 4
<400> 11
   gggtacacca caaagtccc 19
<210> 12
   <211> 21
   <212> DNA
   <213> Bovine herpesvirus 4
<400> 12
   gagcagacca tcatctatag g 21
<210> 13
   <211> 22
   <212> DNA
   <213> Bovine herpesvirus 4
<400> 13
   atggatcaaa gacatcttgt gg 22
<210> 14
   <211> 20
   <212> DNA
   <213> Bovine herpesvirus 4
<400> 14
   ggagagtcct gtctagaacc 20
<210> 15
   <211> 20
   <212> DNA
   <213> Bovine herpesvirus 4
<400> 15
   ggtgagtaat gggaccatgg 20
<210> 16
   <211> 80
   <212> DNA
   <213> Bovine herpesvirus 4
<400> 16
   tctttaaaaa acaaccatag aacatatcat gaagacattc aaaaatattc aaatcaggtg 60
   tcagcactgt cctgctcctt 80
<210> 17
   <211> 84
   <212> DNA
   <213> Bovine herpesvirus 4
<400> 17
   gacatctagt ggtggctcgt atagttacac cagtcttgtg aattttcttt ctgccaaagg 60
   cctgttgaca attaatcatc ggca 84
<210> 18
   <211> 60
   <212> DNA
   <213> Bovine herpesvirus 4
<400> 18
   tctttaaaaa acaaccatag aacatatcat gaagacattc aaaaatattc aaatcaggtg 60
<210> 19
   <211> 60
   <212> DNA
   <213> Bovine herpesvirus 4
<400> 19
   gacatctagt ggtggctcgt atagttacac cagtcttgtg aattttcttt ctgccaaagg 60
<210> 20
   <211> 19
   <212> DNA
   <213> Bovine herpesvirus 4
<400> 20
   gccaacgcat ttggctacc 19
<210> 21
   <211> 19
   <212> DNA
   <213> Bovine herpesvirus 4
<400> 21
   cggcaggcac cagctcttc 19
<210> 22
   <211> 25
   <212> DNA
   <213> Bovine herpesvirus 4
<400> 22
   caaatagttc attagctgcc tctcc 25
<210> 23
   <211> 25
   <212> DNA
   <213> Bovine herpesvirus 4
<400> 23
   tcatcagtaa cagttggaat agtgg 25
<210> 24
   <211> 18
   <212> DNA
   <213> Bovine herpesvirus 4
<400> 24
   gagagtgatt tgtagtgt 18
<210> 25
   <211> 22
   <212> DNA
   <213> Bovine herpesvirus 4
<400> 25
   caaagagatc taggttgtct gg 22
<210> 26
   <211> 22
   <212> DNA
   <213> Bovine herpesvirus 4
<400> 26
   agttaggaca cgttcaccaa cg 22
<210> 27
   <211> 20
   <212> DNA
   <213> Bovine herpesvirus 4
<400> 27
   atgccgggcc gccacctcag 20

## Description

The present invention relates to mutant Bovine Herpesvirus 4 genomes, mutant Bovine Herpesvirus 4 viruses, to mutant Bovine Herpesvirus 4 genomes and viruses carrying a heterologous DNA sequence, cells transfected with mutant Bovine Herpesvirus 4 genomes, cells infected with mutant Bovine Herpesvirus 4 viruses, vaccines and carrier vaccines comprising such mutant Bovine Herpesvirus 4 viruses, methods for the preparation of such mutant Bovine Herpesvirus 4 genomes and such mutant Bovine Herpesvirus 4 viruses and to diagnostic test kits.

Bovine Herpesvirus 4 (BoHV-4) is a member of the subfamily *gammaherpesvirinae,* genus *rhadinovirus,* that is distributed worldwide amongst i.a. ruminants. Isolates have been recovered from cattle, but also from American bison and African buffalo. Isolates have been found also in sheep. Sporadic isolations were reported in lions, cats and owl monkeys.

The virus has been isolated from both healthy and from sick animals. However, although sporadically a possible role for BoHV-4 in respiratory disease has been reported, by far most isolates of the BoHV-4 group fail to induce clinical disease in both cattle and in laboratory animals. Therefore, the pathogenic role of BoHV-4 remains unclear.

BoHV-4, although being a member of the subfamily *gammaherpesvirinae,* differs from other members of this subfamily in that, unlike most other *gammaherpesvirinae,* it replicates in a variety of primary cultures and cell lines of bovine and various other animal species.
In addition, there is no evidence for oncogenicity or growth transformation by BoHV-4.
Moreover, BoHV-4, like all herpesviruses is capable of accommodating significant amounts of foreign genetic material.
Finally, as mentioned above, apparently BoHV-4 does not seem to induce clinical disease.
These characteristics make BoHV-4 attractive for use as a vector virus.
Such use has been suggested i.a. by Donofrio, G. et al., (J. virol. Methods 101: 49-61 (2002), Donofrio, G. et al., (Microb. Infect. 8: 898-904 (2006)), Gillet, L. et al., (J. Gen. Virol. 86: 907-917 (2005)).

A recent example of such use is given by Donofrio, G. et al., (Clinical and Vaccine Immunology 13: 1246-1254 (2006)). In this paper, it is described how BoHV-4 is used for the expression of Glycoprotein D of Bovine Herpesvirus 1, an alphaherpesvirus that is known to be a major pathogen in cattle, that causes significant losses worldwide. BoHV-1 infection is known to cause infectious bovine rhinothracheitis, infectious pustular vulvovaginitis, balanoposthitis, abortion and generalized systemic infection. BoHV-1 is also known to play an important role in the bovine respiratory disease complex commonly referred to as shipping fever. This work is one of the first demonstrations of the use of BoHV-4 as a vector for vaccine purposes and provides a firm basis for BoHV-1-vaccination of cattle by using BoHV-4 as a vector.

There is however one serious drawback:
Gammaherpesviruses like all *Herpesviridae* exhibit two distinct phases in their life cycle; lytic replication characterised by a transcription program where immediate-early (IE), early (E) and late (L) genes are expressed successively; and latency, characterised by the maintenance of the viral genome as a non-integrated episome and the expression of a limited number of viral genes and micro RNAs (Roizman & Pellett, 2007; Weck *et al.,* 1999; Sarid *et al.,* 1998; Cai *et al.,* 2005). Upon reactivation, latency turns into a lytic replication again and re excretion of infectious particles by latently infected subjects.

Therefore, since BoHV-4 has the property to establish latency and to reactivate, this makes wild type strains inappropriate to develop safe recombinant vectors. Indeed, throughout their live, infected animals could reactivate and contaminate naive animals.
Thus, from a vaccine point of view, for BoHV-4 to be used as a vector, the latency of the virus is considered to be a disadvantage.

The gammaherpesvirus human Herpesvirus 8 (HHV-8 also named KSHV for Kaposi's sarcoma-associated Herpesvirus) is the prototype of the *rhadinovirus* genus. During latency, HHV-8 expresses three main open reading frames (ORFs): ORF71, ORF72 and ORF73 (Sarid *et al*.,1998 and 1999). ORF71 and ORF72 encode viral homologues of cellular FLIP (v-FLIP) (Thome *et al.,* 1997) and D cyclin (v-CYC) (Li *et al.,* 1997), respectively. ORF71 and ORF72 are expressed together with ORF73 on a polycistronic mRNA.

ORF73 orthologues encoded by different rhadinoviruses have been studied extensively. These studies revealed that the expression product of ORF73 (pORF73) is a multifunctional protein essential for latency. The multiple functions of the protein rely on several molecular domains (Renne *et al.,* 2001; Fowler et al., 2003; Calderwood *et al.,* 2005). The best-characterised pORF73 is the Latency-Associated Nuclear Antigen 1 (LANA-1) of HHV-8. LANA-1 has been shown to associate with mitotic chromosomes to enable episome tethering during division of latently infected cells and its subsequent partitioning between daughter cells (Ballestas *et al.,* 1999; Barbera *et al.,* 2006; Ye *et al.,* 2004). This crucial role in latency is reinforced by the ability of LANA-1 to modulate cellular pathways implicated in cell growth and survival by targeting transcriptional regulators or co-regulators such as p53, pRB, CBP and sin3A (Friborg *et al.,* 1999; Krithivas *et al.,* 2000; Radkov *et al.,* 2000; Lim *et al.,* 2001). Moreover, LANA-1 prevents reactivation from latency by downregulating the expression of ORF50 that encodes the replication and transcriptional activator (RTA) (Lan *et al.,* 2004, 2005; Lu *et al.,* 2006).

BoHV-4 behaves different in many respects, as summarised above, when compared to other herpesviruses, even within the subfamily *gammaherpesvirinae.* Nevertheless, if any ORF73-orthologue exists in BoHV-4, it could then be possible to find out if such ORF73-orthologue, if existent at all, in BoHV-4 would play a comparable role in latency.

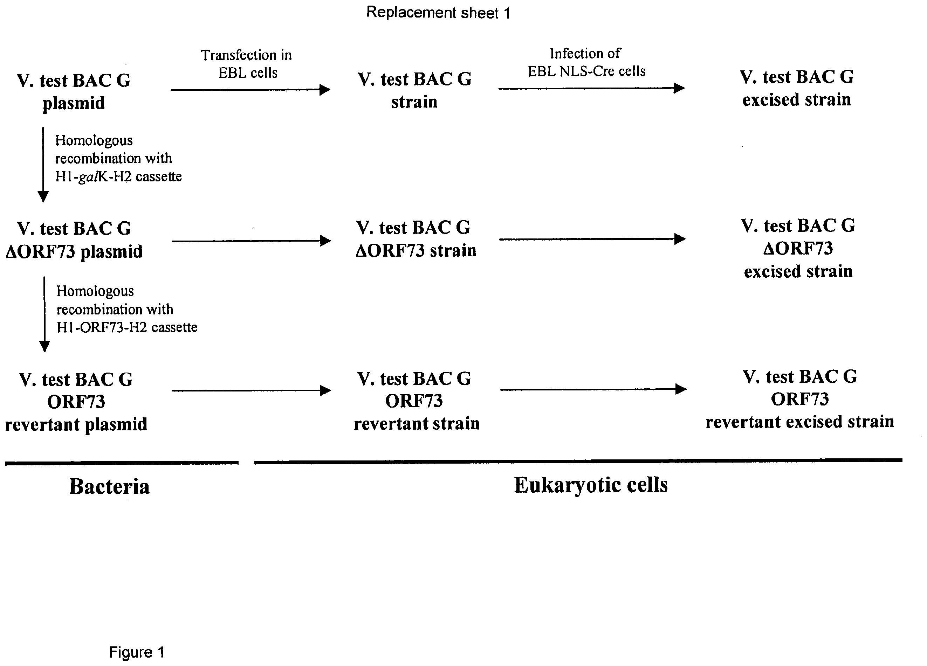

However, the only possibly ORF73-like orthologue gene found in BoHV-4 was considered to be a pseudo gene, for the following reasons:
1) The ORF73 orthologue region of BoHV-4 is the site of multiple recombinations (Dewals B., et al., J. Gen. Virol. 87: 1509-1519 (2006)). This is a characteristic of a pseudo gene, but certainly not of a multi-functional gene that is highly depending on full conservation of its sequence.
2) ORF73 orthologue length variations are observed between various BoHV-4 strains. E.g. a short duplication is observed in the M40 strain. Again this is fully in line with the characteristics of a pseudo gene, but it is highly unusual for a functional, let alone a multi-functional gene.
3) ORF73 of HHV-8 encodes a large and multifunctional protein, that has been shown a) to associate with mitotic chromosomes to enable episome tethering during division of latently infected cells and its subsequent partitioning between daughter cells, b) to modulate cellular pathways implicated in cell growth and survival by targeting transcriptional regulators or co-regulators such as p53, pRB, CBP and sin3A, and c) to prevent reactivation from latency by downregulating the expression of ORF50 that encodes the replication and transcriptional activator.
   Within the rhadinovirus genus, BoHV-4 appeared to encode by far the shortest ORF73 orthologue with a size equivalent to only 22% of ORF73 of HHV-8 (LANA-1) (Fig. 1). BoHV-4 ORF73 consists of only 253 amino acids. Only the 138 N-terminal and the 102 C-terminal amino acid residues exhibit some homology to LANA-1 N- and C-terminal regions, respectively. All other regions are simply missing. Consequently, it was not to be expected that the extremely shortened ORF73 orthologue found in BoHV-4, lacking 78% of the essential structures of LANA-1 including the P-rich domain, the D/E-rich domain, the Q/E-rich domain and the L-zipper, could act as a multifunctional protein having the characteristics of LANA-1.
4) By far most other known ORF73 orthologues are not much smaller in size than LANA-1, or even bigger. Only one relatively small ORF73 orthologue has been described: For murine herpesvirus-68, an ORF73 orthologue has been found. This ORF73 is however still 123% of the size of the BoHV-4 orthologue, and most strikingly, the percentage of amino acid identity is practically negligible: only 25%.

Surprisingly it was found now, that this alleged pseudo gene for unknown reasons plays a role that, with regard to its function in latency, appears to be comparable to that of ORF73 in LANA-1. This gene will for reasons of convenience be further referred to as an ORF73 orthologue.

Moreover, it was surprisingly found that deletion of BoHV-4 ORF73 did not affect the capacity of the virus to replicate *in vitro,* while it prevented latent infection *in vivo.* All together, the results of the present invention show that despite its extremely small size and the striking lack of homology to any known ORF73-orthologue, BoHV-4 ORF73 is a functional homologue of larger rhadinovirus orthologues. The sequence of BoHV-4 ORF73 is available from Genbank/EMBL/DDBJ accession numbers AY847342-AY847348.

Thus, it is one of the merits of the present invention that it provides a BoHV-4 virus from which the ORF73 gene is deleted, thus providing a vector with all advantages of BoHV-4 vector viruses without having the disadvantage of causing latent infection.

The present invention relates to a mutant BoHV-4 genome that has as a characteristic that it does not encode a functional ORF73 protein.

A functional ORF73 protein is a protein that is still capable of causing latency.

The present invention relates to a mutant BoHV-4 virus that it is not capable of making a functional ORF73 protein.

Preferably, the mutant BoHV-4 virus is not capable of making a functional ORF73 protein because it has a mutation in the ORF73 gene.
This relates to mutant BoHV-4 virus according to the invention that has as a characteristic that it has a mutation in the ORF73 gene.

A mutation can be an insertion, a deletion or a frame shift mutation. The easiest way of making a mutation is by means of inserting a stretch of nucleotides or by deleting a part or the whole of the ORF73 coding region.
This relates to mutant BoHV-4 virus wherein said mutation is a deletion or an insertion.

It is known that several genes play a role in the virulence of herpesviruses in general, and in BoHV-4 too. The deletion of genes encoding such virulence factors improves the safety of the virus, because it behaves less virulent than the wild-type virus. Therefore, especially when using the mutant BoHV-4 virus according to the invention as a vaccine or a carrier vaccine, it may be desirable to delete one or more of such virulence factors. Such deletions are for the purpose of this patent herewith defined as second deletions.
This relates to mutant BoHV-4 virus according to the invention that comprises a second deletion.

Preferred second deletions are deletions in the ORF71-gene encoding a v-FLIP, the ORF16-gene encoding a v-Bcl-2 homologue, the Bo10 gene encoding glycoprotein gp80 or the ORF21-gene encoding thymidine kinase.
Therefore, a in an embodiment the mutant BoHV-4 virus according to the invention relates to such viruses having a second deletion in the ORF71-gene encoding a v-FLIP, the ORF16-gene encoding a v-Bcl-2 homologue, the Bo10 gene encoding glycoprotein gp80 or the ORF21 -gene encoding thymidine kinase.

Although the mutant BoHV-4 virus according to the invention can be used for vaccine purposes as such, merely in order to prevent susceptible animals, such as ruminants from BoHV-4 infection, it is most efficiently used as carrier virus for heterologous DNA sequences. In that case, the advantageous characteristics of the mutant BoHV-4 virus according to the invention would be fully used and in addition the virus would e.g. gain additional immunizing or adjuvating properties.
Thus, a further embodiment of the present invention relates to a mutant BoHV-4 virus according to the invention, that comprises a heterologous DNA sequence.

Preferably, such a heterologous DNA sequence encodes an immunogenic protein of another virus or microorganism that is pathogenic to animals that are sensitive to BoHV-4 infection, such as ruminants. The advantage of such a mutant BoHV-4 virus would be that by administrating such virus to such animals, e.g. ruminants, one would be able to protect against BoHV-4, and against that other pathogenic virus or microorganism. And even if there would be no interest in obtaining protection against BoHV-4 infection, such a virus would have all the advantages of a very safe live carrier virus: it is a very safe life BoHV-4 vaccine that at the same time mimics a live vaccine against that other (e.g. ruminant) pathogenic virus or microorganism. Merely as an example: a mutant BoHV-4 virus according to the invention would be a very attractive BoHV-1 gD carrier alternative instead of the BoHV-4 strain as used by Donofrio G., et al., in Clinical and Vaccine Immunology 13: 1246-1254, (2006).
Also, preferably such heterologous DNA sequence encode a cytokine. Cytokines comprise i.a. the interleukins, interferons and tumor necrosis factors. Several cytokines, e.g. interferons are known to play an important role as immune modulators in mammals, e.g. ruminants. Thus it may be advantageous to include a gene encoding this kind of molecule into said virus.

Thus, a preferred form of this embodiment relates to mutant BoHV-4 virus according to the invention comprising a heterologous DNA sequence encoding an immunogenic protein of another virus or microorganism pathogenic to animals that are sensitive to BoHV-4 infection, e.g. ruminants, and/or a cytokine.

The most common and/or important (i.a. ruminant) pathogenic microorganisms and viruses are formed by the group consisting of Bovine Herpesvirus, Bovine Viral Diarrhoea virus, Parainfluenza type 3 virus, Bovine Paramyxovirus, Foot and Mouth Disease virus, *Pasteurella haemolytica,* Bovine Respiratory Syncytial Virus, *Theileria* sp., *Babesia* sp., *Trypanosoma species, Anaplasma* sp., *Neospora caninum, Staphylococcus aureus, Streptococcus agalactiae,* Mycoplasma, *E. coli, Enterobacter, Klebsiella, Citrobacter* and *Streptococcus dysgalactiae.* Therefore, a more preferred form of this embodiment relates to mutant BoHV-4 virus according to the invention wherein that virus or micro-organism is selected from the group consisting of Bovine Herpesvirus, Bovine Viral Diarrhoea virus, Parainfluenza type 3 virus, Bovine Paramyxovirus, Foot and Mouth Disease virus, *Pasteurella haemolytica,* Bovine Respiratory Syncytial Virus, *Theileria* sp., *Babesia* sp., *Trypanosoma species, Anaplasma* sp., *Neospora caninum, Staphylococcus aureus, Streptococcus agalactiae,* Mycoplasma, *E. coli, Enterobacter, Klebsiella, Citrobacter* and *Streptococcus dysgalactiae.*

As said above, mutant BoHV-4 virus according to the invention is not capable of making a functional ORF73 protein. This can i.a. be obtained by making an insertion in the gene. It would therefore be most convenient to insert the heterologous DNA sequence directly into the ORF73 gene. That would avoid the necessity to find a suitable location in the viral genome to introduce the heterologous DNA sequence and would at the same time make the gene encoding ORF73 nonfunctional.

Also from a safety point of view, it is very beneficial to insert the heterologous DNA sequence directly into ORF73: if a recombination occurs between a wild type strain and the vaccine, the recombinant that acquires the heterologous DNA sequence will also acquire the genetic defect of the vaccine and thus not be able to persist as a latent virus.

Thus a most preferred form of this embodiment relates to mutant BoHV-4 virus according to the invention, wherein the heterologous DNA sequence is inserted in the ORF73 gene.

The heterologous DNA sequence encoding the antigen or cytokine is preferably placed under the control of a functional promoter. A functional promoter is a promoter that is recognized in ruminant cells.

A large number of suitable promoters is known in the art, that are recognized for their very efficient level of expression. Such promoters are e.g. the Pseudorabies gX-promoter, the Pseudorabies TK-promoter, the Adenovirus Major Late promoter, the Retroviral Long Terminal Repeat, the SV40 Early and Late promoters, the Mouse Cytomegalovirus immediate early (MCMViel) promoter, the Mouse Cytomegalovirus early (MCMVe1) promoter, the Human Cytomegalovirus immediate early (HCWie1) promoter, and the BHV-gE promoter. All these promoters have been described and are known in the art for many years. Merely as an example in Donofrio G., et al. describe the use of the Human Cytomegalovirus enhancer-promoter for the expression of gD of BoHV-1. (Donofrio G., et al., Clinical and Vaccine Immunology 13: 1246-1254, (2006))

As extensively motivated above, the mutant BoHV-4 viruses according to the invention are very suitable as vaccine viruses or carrier vaccine viruses.
Therefore, again another embodiment of the present invention relates to vaccines for combating BoHV-4 infection in animals susceptible for BoHV-4 such as ruminants, wherein such vaccines comprise a mutant BoHV-4 genome according to the invention or a mutant BoHV-4 virus according to the invention, and a pharmaceutically acceptable carrier.

The pharmaceutically acceptable carrier can be as simple as water or a buffer. The pharmaceutically acceptable carrier may also comprise stabilizers. It can also comprise an adjuvant, or it can in itself be an adjuvant.

The vaccine can be safely stored for long periods of time when it is freeze-dried. Therefore, the vaccine according to the invention is preferably in a freeze-dried form.

Still another embodiment relates to mutant BoHV-4 carrier vaccines that comprise a BoHV-4 virus according to the invention comprising a heterologous DNA and a pharmaceutically acceptable carrier.

Again another embodiment of the invention relates to a mutant BoHV-4 genome according to the invention or mutant BoHV-4 virus according to the invention for use in a vaccine.

Still another embodiment of the invention relates to the use of a mutant BoHV-4 genome according to the invention or a mutant BoHV-4 virus according to the invention for the manufacture of a vaccine for the protection of susceptible animals such as ruminants against BoHV-4 infection.

And again another embodiment of the invention relates to the use of a mutant BoHV-4 genome according to the invention or a mutant BoHV-4 virus according to the invention for the manufacture of a BoHV-4 carrier vaccine.

As follows i.a. from the Examples, it is possible to make the necessary mutant BoHV-4 genome in vitro. If a cell is transfected with this genome, this leads to the generation of progeny virus. Thus, another embodiment of the present invention relates to a cell comprising a mutant BoHV-4 genome according to the invention.

The progeny virus in itself is fully infective, and therefore, that progeny virus can be propagated on susceptible cells, such as e.g. MDBK cells.
Therefore, another embodiment of the present invention relates to a cell comprising a mutant BoHV-4 virus according to the invention.

For administration to animals, the BoHV-4 mutant according to the present invention can be given inter alia intranasally, intradermally, subcutaneously or intramuscularly. The routes and methods for vaccination can be the same as for other live attenuated BHV-viruses, more specifically BoHV-4 vaccines.

The useful effective amount to be administered will vary depending on the age, weight, mode of administration and type of pathogen against which vaccination is sought.

For most applications, a very suitable dosage range is between about 10^{3.0} - 10^{7.0} pfu/animal.

Another embodiment of the present invention relates to methods for the production of a mutant BoHV-4 genome according to the invention or a mutant BoHV-4 virus according to the invention, wherein that method comprises the step of mutating the ORF73 gene so that it no longer encodes a functional ORF73 protein.

An interesting effect of the fact that mutant BoHV-4 according to the invention can carry a heterologous gene, is that it can thus be used as a marker vaccine. A marker vaccine allows to discriminate between field viruses and vaccine viruses on the basis of differences in antibody panels raised against wild-type BoHV-4 and mutant BoHV-4 virus according to the invention when carrying an additional gene.

Sera from mammals infected with a mutant BoHV-4 strain according to the invention that carries a heterologous gene will react with immunogenic BoHV-4-proteins and with an immunogenic protein encoded by a heterologous gene inserted in mutant BoHV-4 according to the invention. Sera from mammals infected with an BoHV-4 field strain however will only react with immunogenic BoHV-4-proteins. Tests based upon purified protein encoded by the heterologous gene directly show the presence or absence of antibodies against the protein encoded by the heterologous gene. Therefore, mutant BoHV-4 viruses according to the present invention that carry a heterologous gene turn out to be very suitable marker vaccines, i.e. vaccines that can be serologically discriminated from a field strain. They can be discriminated by using specific diagnostic tests,
for differentiating BoHV-4 field-virus infected mammals from mammals vaccinated with a vaccine comprising mutant BoHV-4 according to the invention and carrying a heterologous gene. Such tests comprise the testing of serum for the absence or presence of antibodies against the protein encoded by the heterologous gene.

A diagnostic test kit for the differentiation between animals vaccinated with mutant BoHV-4 strains according to the invention and carrying a heterologous gene, and animals infected with BoHV-4 field strains can comprise e.g. a simple blocking-ELISA test in which a mutant BoHV-4 strain according to the invention and carrying a heterologous gene is coated to the wall of a microtiter-plate. Such a plate is then incubated with serum of the animal to be tested. Subsequently, the plate is incubated with a specific and labeled antiserum against the heterologous protein. If the labeled antiserum reacts with the coated virus, i.e. it is not blocked by the antiserum of the animal to be tested, this shows that that antiserum does not contain antibodies against the heterologous protein. It can then be concluded that the animal was infected by a field strain.

Alternatively, such a diagnostic test kit can comprise an ELISA-test in which possibly purified heterologous protein is coated to the wall of separate wells of an ELISA-plate. Incubation with serum from mammals to be tested, followed by e.g. incubation with a labeled antibody against the heterologous protein can then reveal the presence or absence of antibodies against the heterologous protein in the respective wells. Another example of a diagnostic test system is e.g. the incubation of a Western blot comprising the heterologous protein with serum of mammals to be tested, followed by analysis of the blot.
Detection of specific anti-heterologous protein antibodies indicates that the tested mammal has been vaccinated with a mutant BoHV-4 strain according to the invention and carrying a heterologous gene, whereas the lack of antibody-reaction with the heterologous protein band indicates that the mammal has been infected with a BoHV-4 field strain.

Another approach for using mutant BoHV-4 virus according to the invention in marker vaccines is to discriminate between field viruses and vaccine viruses on the basis of differences in reactions in PCR tests between wild-type BoHV-4 and mutant BoHV-4 virus according to the invention. Such a test in a very basic form could comprise a first set of PCR-primers against any wild-type BoHV-4 sequence and a second set of PCR-primers against a region of ORF73 that has been deleted from the mutant BoHV-4 virus according to the invention.
If blood or tissue from an animal shows a reaction with both the first and the second primer set, this indicates that the animal was infected with a field virus. If blood or tissue from an animal however only shows a reaction with the first and not with the second primer set, this indicates that the animal was vaccinated with a mutant BoHV-4 virus according to the invention.

Merely to exemplify aspects of the invention, the Examples below will explain (without being restrictive in any way) various aspects of the invention in more detail.

### EXAMPLES

### EXAMPLE 1.

**Computational analysis.** Protein sequences were analysed with ClustalX sequence alignment program (Thompson *et al.,* 1997).

**Cells and Viruses.** Madin-Darby bovine kidney cell line (MDBK; ATCC CCL-22) was cultured in minimum essential medium (MEM, Invitrogen) containing 5% fetal calf serum (FCS, Harlan). Embryonic bovine lung (EBL, German collection of micro-organisms and cell culture DSMZ ACC192), EBL stably expressing the Cre recombinase (EBL-NLS-Cre) (Gillet *et al.,* 2005) and bovine macrophage (BOMAC) (Stabel & Stabel, 1995) cell lines were cultured in Dulbecco's modified Eagle medium (DMEM, Invitrogen) containing 10% FCS. The BoHV-4 V. test strain and the V. test BAC G derived bacterial artificial chromosome (BAC) clone were described elsewhere (Gillet *et al.,* 2005).

**RT-PCR, determination of kinetic class of transcription.** For the determination of kinetic class of transcription, cycloheximide (CHX, 100µg/ml, Sigma) or phosphonoacetic acid (PAA, 300 µg/ml, Sigma) were added to the culture medium at the time of infection to inhibit *de novo* protein synthesis or viral DNA polymerase, respectively. 24 h post infection (p.i.) of MDBK cells (multiplicity of infection (MOI) of 1 plaque forming unit (PFU)/cell), cytoplasmic RNA was extracted using the RNeasy Mini Kit (Qiagen) with on-column DNase I digestion. Reverse transcription and PCR reactions were performed using the SuperScript III One-step RT-PCR System with Platinium *Taq* DNA polymerase Kit (Invitrogen). Gene-specific primers (Table S1) were used to detect viral mRNAs. The absence of DNA template was verified by replacing the RT/*Taq* mix by the *Taq* DNA polymerase only.

**Northern blotting.** MDBK cells were mock-infected or infected with BoHV-4 V. test strain at a MOI of 4 PFU/cell. After an incubation period of 90 min, cells were washed with PBS and then overlaid with MEM containing 5% FCS. Cells were scrapped at 4, 8 and 24h p.i.. RNA was then purified from the cells and analysed by northern blotting according to standard protocol (Sambrook & Russell, 2001). The entire ORF73 was used as probe to detect the RNAs encoding ORF73.

**Production of BoHV-4 ORF73 recombinant strains.** BoHV-4 recombinants were produced using BAC cloning and prokaryotic recombination technologies as described before (Gillet *et al.,* 2005). The V. test BAC G plasmid was used as parental plasmid (Gillet *et al.,* 2005). The V. test BAC G ΔORF73 and the V. test BAC G ORF73 revertant plasmids were produced using a two step galactokinase (*gal*K) positive/negative selection in bacteria (Warming *et al.,* 2005, Fig. 1). The first recombination process (*galK* positive selection) consisted to replace ORF73 by the *galK* gene resulting in the V. test BAC G ΔORF73 plasmid. Recombination was achieved using the H1-*gal*K-H2 cassette (Fig. 1). It consisted of the *gal*K gene flanked by 60-bp sequences corresponding to ORF73 flanking regions. This cassette was produced by PCR using p*gal*K vector (Warming *et al.,* 2005) as template and *gal*K-H2endfw and *gal*K-H1 startrev as forward and reverse primers, respectively (Table S1). The second recombination process (*gal*K negative selection) consisted to restore ORF73 to generate a revertant plasmid. Recombination was achieved using the H1-ORF73-H2 cassette (Fig. 1), consisting of ORF73 flanked by 60-bp sequences corresponding to ORF73 flanking regions. This cassette was produced by PCR using BoHV-4 V. test genome as template and H2endfw and H1startrev as forward and reverse primers, respectively (Table S1). Reconstitution of infectious virus from BAC plasmids was obtained by transfection in EBL cells. To excise the BAC cassette, reconstituted viruses were propagated in EBL-NLS-Cre cells expressing Cre recombinase to generate the corresponding excised strain (Fig. 1).

**Virus purification.** Virus grown on MDBK cells was semi-purified as described previously (Vanderplasschen *et al.,* 1993). Briefly, after removal of the cell debris, the virus present in the cell supernatant was pelleted by ultracentrifugation through a 30% (w/v) sucrose cushion.

**Southern blotting.** Southern blot analysis was performed as described previously using ORF73 and *gal*K cassette as probes (Costes *et al.,* 2008).

**Plaque size assay.** MDBK cells were infected at a MOI of 0.5 PFU/cell. After an incubation period of 90 min, cells were washed with PBS and then overlaid with MEM containing 5% FCS and 0.6% (w/v) carboxymethylcellulose (CMC, Sigma) in order to obtain isolated plaques as described elsewhere (Vanderplasschen *et al.,* 1993). At successive intervals after infection, plaques were stained by indirect immunofluorescent staining (see below) and observed by epifluorescent microscopy.

**Multi-step growth curves.** Triplicate cultures of MDBK cells were infected at a MOI of 0.5 PFU/cell. After an incubation period of 90 min, cells were washed with PBS and then overlaid with MEM containing 5% FCS. Supernatant of infected cultures was harvested at successive intervals after infection and the amount of infectious virus was determined by plaque assay on MDBK cells as described previously (Vanderplasschen *et al.,* 1993).

**Episome persistence assay.** BOMAC cells were infected at a MOI of 0.5 PFU/cell. After an incubation period of 90 min, cells were washed with PBS and then overlaid with MEM containing 5% FCS. The cells were then passaged every other day (1:2 split ratio) for 20 days. At each passage, a fraction of the cells was fixed in PBS containing 4% (w/v) paraformaldehyde (Merck) at 4°C for 30 min. Fixed cells were then washed with PBS, resuspended in the same buffer and analysed by flow cytometry for EGFP protein expression (EGFP is encoded by the BAC cassette).

**Animals.** Specific-pathogen-free New-Zealand white rabbits were housed individually throughout this study. Four groups were used, each comprising three rabbits. Animals from group 1 were mock-infected intravenously with PBS. Animals from groups 2, 3 and 4 were inoculated intravenously with 10⁸ PFU of V. test BAC G excised, V. test BAC G ΔORF73 excised and V. test BAC G ORF73 revertant excised strains, respectively. At the end of the experiment, rabbits were euthanized and a necropsy examination was performed during which the spleen was collected.

**Isolation of peripheral blood mononuclear cells and preparation of spleen cell suspension.** Blood samples were collected and peripheral blood mononuclear cells (PBMC) were separated by Ficoll (Ficoll-Paque Plus, GE Healthcare) density gradient as described previously (Dewals *et al.,* 2008). Immediately after euthanasia, spleen was removed and half-part of it was homogenized using a tissue grinder (VWR), passed through a stainless steel sieve and washed in FCS-free MEM before further analyses.

**Viral genome detection by Real time-PCR.** DNA was purified from the spleen and PBMC using the QIAamp DNA Mini kit (Qiagen). Real-time PCR was performed as described elsewhere (Boudry *et al.,* 2007). A 103 bp fragment corresponding to BoHV-4 ORF8 was amplified with the forward primer 8startfw and the reverse primer 8middlerev (Table S1) in the presence of the fluorescent probe 5'-FAM-AACACGTCAACA AGCAAGCCATCCACTG-TAMRA-3'. Purified BoHV-4 V. test genome was used to establish standard curves. PCR amplifications and fluorescence reactions were carried out in a iCycler system (Bio-Rad) under the following conditions: initial activation of the *Taq* polymerase (Bio-Rad) at 94°C for 5 min followed by 50 cycles at 94°C for 1 min, 50 cycles at 51°C for 30 sec and 50 cycles at 72°C for 1 min. For cellular quantitative amplification, a 178 bp fragment of the rabbit beta-globin gene was used (Genbank accession no. V00882) and amplified as described earlier (Boudry *et al.,* 2007).

**Infectious centre assay.** Viral detection in spleen cell suspension was assayed by infectious centre assay (ICA) as follows. 5.10⁵ MDBK cells grown in 6 well cluster dishes (Becton Dickinson) were co-cultured for 4 days at 37°C with 5.10⁶, 5.10⁵ or 5.10⁴ spleen cells in MEM containing 5% FCS, 2% PS, 0.6% CMC and 5.10⁻⁵M of β-mercaptoethanol (Merck). Cells were then fixed and stained for indirect immunofluorescent detection of viral antigen as described below.

**Quantification of anti-BoHV-4 antibodies by ELISA.** Nunc Maxisorp ELISA plates (Nalge Nunc) were coated for 18 h at 37°C with 0.1% Tween 20 (v/v) - disrupted BoHV-4 virions (2.10⁶ PFU/well), blocked in PBS containing 0.1% Tween-20 (v/v) and 3% BSA (w/v), and incubated with rabbit sera (diluted 1/300 in PBS containing 0.1% Tween-20 (v/v) and 3% BSA (w/v)). Bound antibodies were detected with Alkaline Phosphatase conjugated goat anti-rabbit Ig polyclonal antibody (Sigma). Washing were performed with PBS containing 0.1% Tween-20 (v/v) and 3% BSA (w/v). Nitrophenylphosphatate (Sigma) was used as substrate and absorbance was read at 405nm using a Benchmark ELISA plate reader (Thermo).

**Indirect immunofluorescent staining.** Indirect immunofluorescent staining was performed as described previously (Gillet *et al.* 2005). Mouse monoclonal antibody (Mab) 35 (diluted 1:100) raised against the BoHV-4 glycoprotein complex gp6/gp10/gp17 and Alexa Fluor 568 goat anti-mouse IgG (H+L) (Invitrogen, 2µg ml⁻¹) were used as primary and secondary antibodies, respectively.

**Microscopy analysis.** Samples were examined using an epifluorescence microscope TE2000-S Nikon and a Leica DC300F CCD camera system.

**Flow cytometry.** Flow cytometry acquisitions and analyses were performed using a three lasers Becton Dickinson fluorescence-activated cell sorter (FACSAria).

**Statistical analysis.** The linear model y = µ + groupᵢ + timeⱼ + groupᵢ*timeⱼ + experimentₖ + errorᵢⱼₖₗ was used to analyse viral multi-step growth curves and rabbit anti-BoHV-4 humoral responses. For multi-step growth curves comparison, the experiment factor was not significant and was therefore not included in the model. For analysis of anti-BoHV-4 humoral responses, correlation between successive measurements made on the same animal was taken into account using compound symmetry structure. Anova-2 test was used to determine the significance of the data obtained with the episome persistence assay (P<0.0005).

### RESULTS

### BoHV-4 ORF73 is expressed as an IE gene.

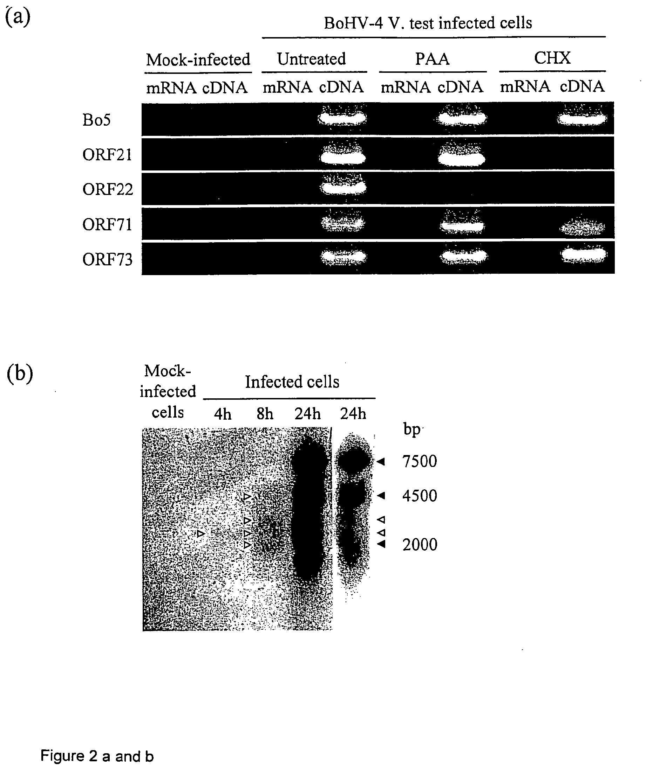

To determine if BoHV-4 ORF73 is a bona fide gene, it was determined whether it is transcribed during BoHV-4 infection. RT-PCR was performed using first-strand cDNA made from BoHV-4-infected and mock-infected MDBK cells (Fig. 2a). In contrast to mock-infected cells, cDNA from infected cells generated a 762 bp PCR product (Fig. 2a) corresponding to ORF73 as determined by sequencing. When RT was omitted from the reactions, no PCR product was detected, indicating that it did not result from amplification of contaminant viral DNA. All together, these data demonstrated that ORF73 is transcribed during BoHV-4 replication *in vitro.* A similar conclusion was reached for ORF71 based on the data presented in Fig. 2a. Next, CHX and PAA were used to identify ORF73 and ORF71 kinetic class of transcription. This experiment revealed that neither CHX nor PAA inhibited ORF73 and ORF71 expression suggesting that both ORFs are expressed as IE genes. Bo5, ORF21 and ORF22 were used as controls in this experiment; the results presented in Fig. 2a confirmed that they are IE, E and L genes, respectively. The absence of contaminant viral DNA in the mRNA preparations was confirmed by the absence of a PCR product when the reverse transcriptase was omitted from the reactions (Fig. 2a). The expression of BoHV-4 ORF73 as an IE gene is consistent with earlier reports describing the expression of ORF73 orthologues encoded by rhadinoviruses early after infection (Ebrahimi *et al.,* 2003; Martinez-Guzman *et al.,* 2003; Sarid *et al.,* 1999).

**BoHV-4 ORF73 is expressed as polycistronic mRNAs encompassing ORF71.** To characterise ORF73 transcript(s), Northern blot analysis was performed on total RNA extracted from BoHV-4-infected and mock-infected MDBK cells using full-length ORF73 as probe (Fig. 2b). A weak band of approximately 2.5 kb was detected as early as 4h p.i. (Fig. 2b). At 8h p.i., four bands were detected with approximate size of 2.0, 2.5, 3.0 and 4.5 kb (Fig. 2b). One additional band of approximately 7.5 kb was detected 24h p.i. (Fig. 2b). These results suggest that ORF73 expression involves transcripts of different sizes. No signal was detected in RNA extracted from mock-infected cells (Fig. 2b).

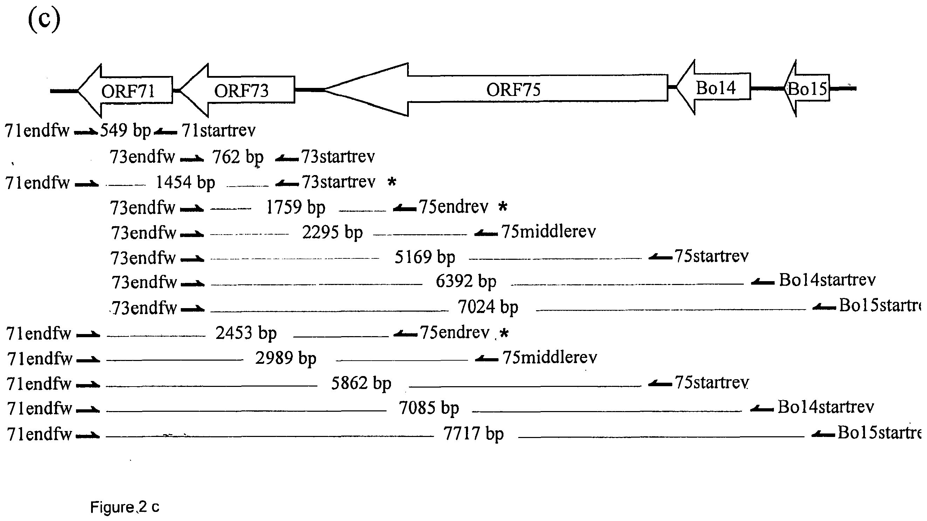

ORF71 is expressed as a polycistronic RNA encompassing at least ORF73. The polyadenylation signal (AATAAA) of the transcript is located 104 bp downstream of ORF71 stop codon. RT-PCR reactions were performed on cytoplasmic RNA extracted from BoHV-4-infected MDBK cells in order to further characterise polycistronic RNA molecules encoding ORF71 and ORF73 (Fig. 2, panels c and d). PCR products corresponding to ORF71- 73 (1454 bp), ORF73- end of ORF75 (1759 bp) and ORF71- end of ORF75 (2453 bp) were amplified (Fig. 2d). None of the PCR reactions involving primers hybridizing upstream of primer 75 endrev generated a product (Fig. 2d), while all reactions generated the expected products when performed on control viral DNA. Absence of contaminant viral DNA was confirmed by the absence of PCR product when RT was omitted from the reactions. All together, the data presented above suggest that ORF71 and ORF73 are expressed as a bicistronic mRNA. At least two mRNAs were identified by combined Northern blot/RT-PCR approach; one encompassing both ORF71 and ORF73, the second encompassing ORF71, ORF73 and part of ORF75 (Fig. 2b and d). Longer RNAs were identified by Northern blot approach but not by RT-PCR. These molecules could represent pre-mRNAs.

**Production and characterisation of BoHV-4 ORF73 recombinants.** In order to investigate the importance of ORF73 in virus replication *in vitro* and the biology of the infection *in vivo,* a BoHV-4 strain deleted for ORF73 and a derived revertant strain were produced (Fig. 1). The V. test BAC G plasmid was used as parental plasmid to generate the V. test BAC G ΔORF73 plasmid in which the entire ORF73 had been replaced by a *gal*K cassette as described in the Methods. Then, a revertant plasmid was generated. Infectious viruses were efficiently reconstituted by transfection of BAC plasmids into permissive cells. Finally, reconstituted infectious viruses were propagated in EBL-NLS-Cre cells to generate BAC-excised strains (Fig. 1). The molecular structures of the recombinant strains produced were confirmed by a combined *Hin*dIII restriction endonuclease and Southern blot approach (Fig. 3) and by sequencing of the regions used to target recombination. Moreover, the absence of polar effect of ORF73 deletion on the expression of ORF71 was demonstrated by RT-PCR. All recombinants tested expressed ORF71 comparably. Finally, presence or absence of the BAC cassette was monitored by analysis of EGFP expression by viral plaques (Fig. 4a). All approaches confirmed that the recombinants produced have the correct molecular structure.

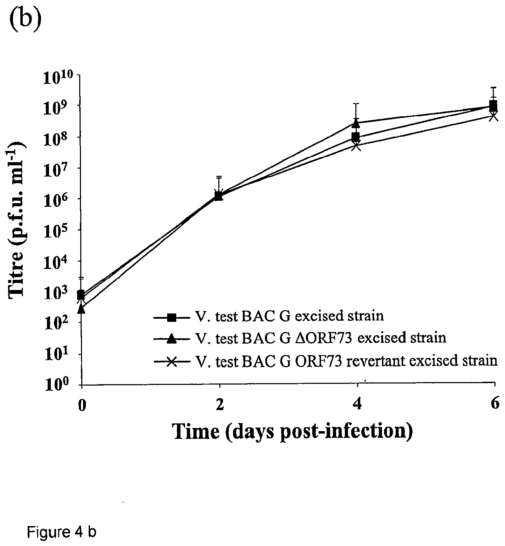

**ORF73 deletion does not affect viral growth *in vitro.*** In order to address the role of ORF73 in BoHV-4 growth *in vitro,* V. test BAC G, V. test BAC G ΔORF73 and V. test BAC G ORF73 revertant excised strains were compared using multi-step growth and plaque size assays (Fig. 4). Corresponding non excised strains were also compared. All viruses tested exhibited similar growth curves (p≤0.05) and comparable plaque sizes. Taken together, these results indicate that ORF73 deletion does not affect BoHV-4 replication *in vitro.*

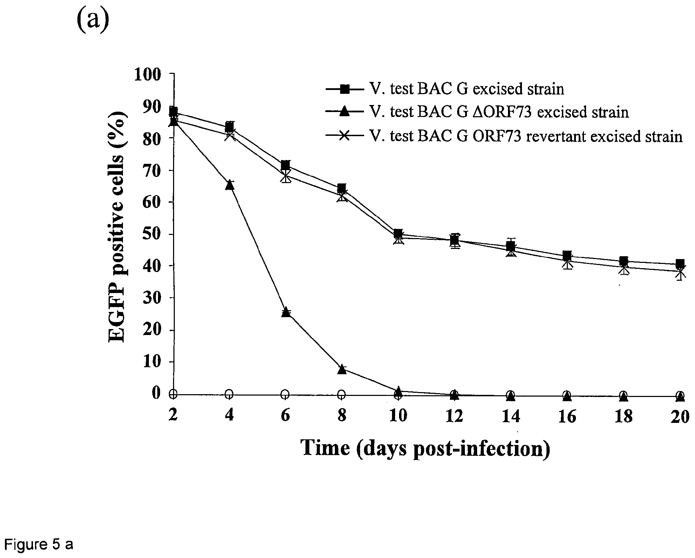

**ORF73 deletion affects viral persistence *in vitro* in a "latency-like" model.** BOMAC cells have been shown to support a BoHV-4 non replicative persistent infection mimicking latency (Donofrio & van Santen, 2001). Using this "latency-like" model, it was investigated whether ORF73 deletion affects the persistence of BoHV-4 infection in BOMAC cells. BOMAC cells were infected with the V. test BAC G, V. test BAC G ΔORF73 and V. test BAC G ORF73 revertant strains and viral persistence was monitored over time by flow cytometry quantification of EGFP expressing cells (Fig. 5a). Independently of the recombinants, about 90% of the cells were positive for EGFP expression on day 2 p.i.. For all three recombinants tested, the percentage of EGFP positive cells decreased gradually over time. However, while comparable data were obtained for V. test BAC G and V. test BAC G ORF73 revertant strains, a faster decay of positive cells was observed for cells infected with V. test BAC G ΔORF73 strain (Fig. 5a). For example, on day 10 p.i. while V. test BAC G and V. test BAC G ORF73 revertant infected cultures contained approximately 50% of EGFP positive cells, the percentage of EGFP positive cells in BOMAC cells infected with V. test BAC G ΔORF73 strain was close to 0.

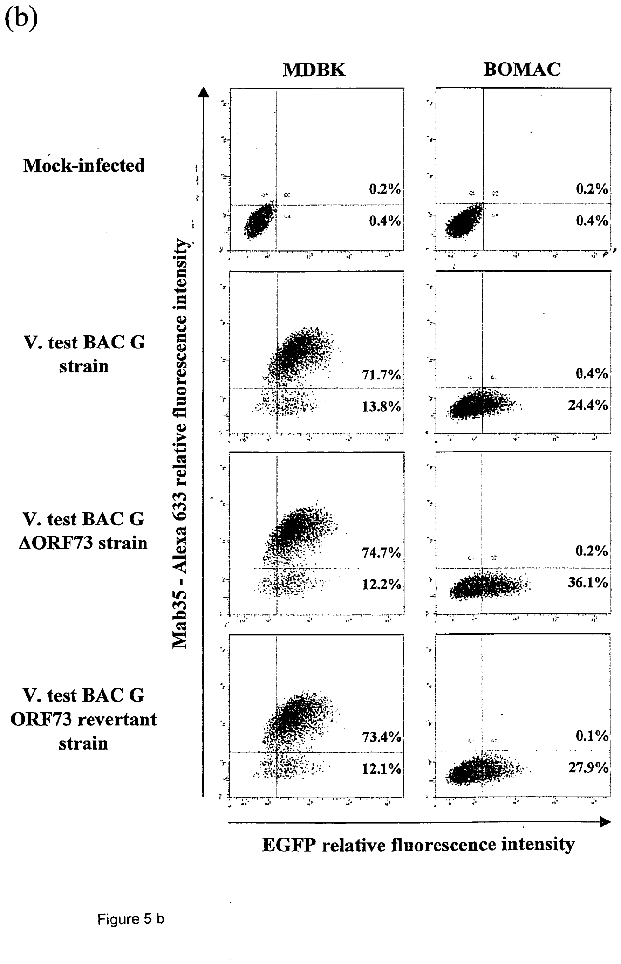

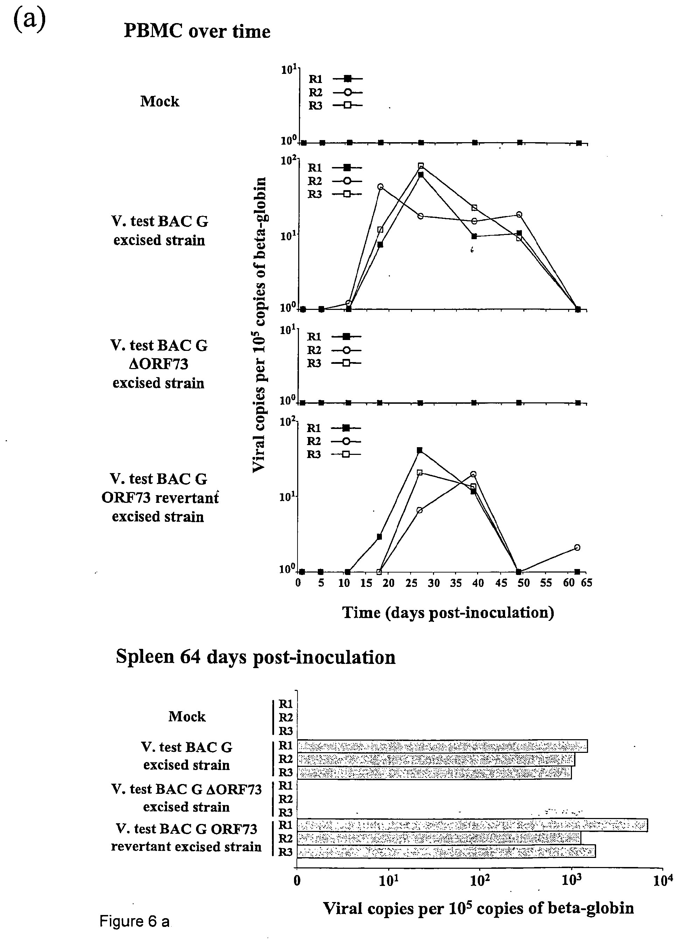

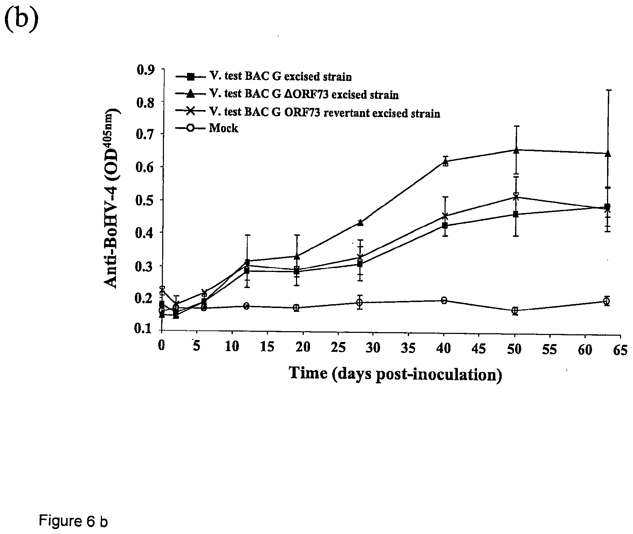

At least two hypotheses that are not mutually exclusive could explain the faster decay of EGFP positive cells in V. test BAC G ΔORF73 infected culture in comparison to V. test BAC G and V. test BAC G ORF73. Firstly, it is likely that the deletion of ORF73 impaired the persistence of the viral episome in the culture through cell division. Secondly, it is possible that the absence of ORF73 could induce lytic replication (by release of the inhibition on ORF50) of BoHV-4 in BOMAC cells and consequently eradication of infected cells from the culture. To test the latter hypothesis, BOMAC cells were infected with V. test BAC G, V. test BAC G ΔORF73 and V. test BAC G ORF73 revertant strains, then analysed 48 h p.i. by flow cytometry for expression of the structural protein gB. The data presented in Fig. 5b demonstrate that independently of the viral strain used for the infection, no expression of BoHV-4 gB could be detected in infected BOMAC cells (Fig. 5b, right column). In contrast, infection of MDBK cells used as positive control led to a large proportion of gB expressing cells (Fig. 5b, left column). All recombinants tested led to comparable percentages of gB expressing cells. All together, the results presented above support the fact that BoHV-4 ORF73 plays a role in latency and justified the completion of *in vivo* experiments.
**BoHV-4 ORF73 is essential for viral persistence *in vivo.*** To investigate the importance of BoHV-4 ORF73 during latency *in vivo,* the rabbit model was used. Rabbits were inoculated with the V. test BAC G, V. test BAC G ΔORF73 and V. test BAC G ORF73 revertant excised strains (Fig. 6a and b). Independently of the groups, all rabbits remained healthy for the course of the experiment. None of the rabbits showed clinical symptoms or lesions throughout the experiment. No lesion was observed at necropsy 64 days post-inoculation. To address viral persistence, real-time PCR were performed on DNA extracted from PBMC over time and from the spleen 64 days post-inoculation (Fig. 6a). After an eclipse phase of 12 days, viral copies ranging from 10¹ to 10² viral copies per 10⁵ copies of beta-globin were detected in PBMC from all rabbits infected with the V. test BAC G and V. test BAC G ORF73 revertant excised strains (Fig. 6a). Interestingly, none of the rabbits infected with the V. test BAC G ΔORF73 excised strain had detectable viral genome in PBMC throughout the experiment (Fig. 6a). Real-time PCR performed on the spleen confirmed the essential role of ORF73 for viral persistence *in vivo.* Indeed, while rabbits infected with the V. test BAC G and V. test BAC G ORF73 revertant excised strains contained at least 10³ viral copies per 10⁵ cellular beta-globin copies, none of the rabbits infected with the V. test BAC G ΔORF73 excised strain exhibited detectable amount of viral genome (Fig. 6a). To confirm these real-time PCR results, viral isolations were attempted by co-cultivation of spleen cells with permissive cells as described in Methods. Viral plaques were observed for the 3 rabbits of the group infected with the V. test BAC G excised strain and for 2 of the 3 rabbits infected with the V. test BAC G ORF73 revertant excised strain. In contrast, no virus was isolated from spleen cells of rabbits infected with V. test BAC G ΔORF73 excised strain. Viral isolation assays performed with the same sources of spleen cells, but frozen-thawed before the assay, did not led to viral isolation. Taken together, the results presented above demonstrate that ORF73 is essential for viral latency.

Finally, the humoral immune response induced by the ORF73 deleted strain was compared to those observed with the wild type parental and the revertant strains (Fig. 6b). Independently of the viral strain used for the inoculation, anti-BoHV-4 antibodies were detected as early as 6 days post-inoculation. All infected rabbits exhibited comparable humoral immune responses. The statistical test used to analyse the data suggested that for some time points, rabbits infected with V. test BAC G ΔORF73 excised strain exhibited a higher response (p≤ 0.05) (Fig. 6b) when compared to the V. test BAC G and V. test BAC G ORF73 revertant excised strains. This statistical difference was not observed when the Student's *t*-test was used. However, these data demonstrate that the immune response raised against the ORF73 deleted recombinant is at least comparable to the responses induced by the wild type and the revertant strains. Absence of response in the mock-infected group confirmed absence of cross-contamination between the groups (Fig. 6b).

Several features make BoHV-4 a good candidate as a gene delivery vector and previous studies showed evidence that BoHV-4 can successfully be used to transduce expression of foreign proteins (Gillet *et al.,* 2005; Donofrio *et al.,* 2006). BoHV-4 (*i*) possesses a less complex genome than other herpesviruses (Zimmermann *et al.,* 2001), (*ii*) can accommodate large amounts of foreign genetic material (Gillet *et al.,* 2005), (*iii*) in contrast to most gammaherpesviruses, is able to replicate in a broad range of host species both *in vitro* and *in vivo* (Thiry *et al.,* 1992), and (*iv*) BoHV-4 exhibits limited or no pathogenicity in natural and experimental hosts (Thiry *et al.,* 1992). However, the property of BoHV-4 to establish latency and to reactivate makes wild type strains inappropriate to develop safe recombinant vectors. Indeed, throughout their live, infected animals could reactivate and contaminate naive animals. The results of the present study demonstrate that ORF73 deletion abolishes the property of BoHV-4 to establish persistent infection at least in a laboratory animal model (Fig. 6). Consequently, BoHV-4 ORF73 deleted strains represent excellent basis vectors for the development of BoHV-4 vectors in vaccinology.

In conclusion, the results of the present study demonstrate that despite its very small size, BoHV-4 ORF73 is a functional homologue of larger rhadinovirus ORF73 orthologues that is essential for latency *in vivo.* BoHV-4 ORF73 deleted strains represent excellent basis vectors for the development of BoHV-4 vectors in vaccinology.

**Table S1. Oligonucleotides used for PCR amplification**

| Primers | Sequence | Coordinates according to Genbank accession no. NC_002665 |
|---|---|---|
| Bo5endfw | 5'-TCATGTCCTGAGTGGGTCTATG-3' | 19414-19435 |
| Bo5startrev | 5'-GCTACAGAAAATGGCCAGTAAAG-3' | 20541-20563 |
| 21middlefw | 5'-CATGGAGAGGCCATCAGGAG-3' | 29044-29063 |
| 21endrev | 5'-CTTAACCACAGAACTCCTTTGC-3' | 29521-29542 |
| 22middlefw | 5'-CCGGGTGAAACAAGTTCCTG-3' | 31114-31133 |
| 22endrev | 5'-GGTCAGAGAACATATGATAACATC-3' | 31654-31675 |
| 71endfw | 5'-CCAAGCTTCTAGAGAGTGATTTGTAGTGT-3' | 98883-98903 |
| 71 startrev | 5'-AAGGATCC*G*ATGGTTACCAGGGATGTGTTG-3' | 99411-99431 |
| 73endfw | 5'-TCACTGATCAACGGGTATTCC-3' | 99576-99596 |
| 73 startrev | 5'-ATGCCGGGCCGCCACCTC-3' | 100320-100337 |
| 75endrev | 5'-GGGTACACCACAAAGTCCC-3' | 101317-101335 |
| 75middlerev | 5'-GAGCAGACCATCATCTATAGG-3' | 101851-101871 |
| 75startrev | 5'-ATGGATCAAAGACATCTTGTGG-3' | 104724-104745 |
| Bo14startrev | 5'-GGAGAGTCCTGTCTAGAACC-3' | 105949-105968 |
| Bo15startrev | 5'-GGTGAGTAATGGGACCATGG-3' | 106581-106600 |
| *gal*K-H2endfw | | 99517-99575 |
| *gal*K-H1startrev | | 100338-100397 |
| H2endfw | | 99517-99575 |
| H1 startrev | | 100338-100397 |
| *gal*Kstartfw | 5'-GCCAACGCATTTGGCTACC-3' | |
| *gal*Kendrev | 5'-CGGCAGGCACCAGCTCTTC-3' | |
| 8startfw | 5'-CAAATAGTTCATTAGCTGCCTCTCC-3' | 11571-11595 |
| 8middlerev | 5'- TCATCAGTAACAGTTGGAATAGTGG -3' | 11649-11673 |

Underlined: extrabases added. In bold: mutation specific to the BoHV-4 strain used in the present study (V. test, Genbank accession no. Z79633).

**Supplementary Table S2. GenBank accession numbers of BoHV-4 sequenced regions**

| **Strain** | **ORF 16** | **Bo10** | **ORF 71** | **intergenic region 71-73** | **ORF 73** | **Bo17** |
|---|---|---|---|---|---|---|
| V. test | AY847335 | Z84818 | Z46385* | Z46385* | Z46385* | AF321105*† |
| LVR 140 | AY847332 | AY847310 | AY847339 | AY847325 | AY847346 | AF485330*‡ |
| MOVAR | AY847334 | AYS47305 | AY847341 | AY847327 | AY847348 | AF465331*‡ |
| DN599 | AY847331 | AY847309 | U23760* | U23760* | AY847345 | AF465332*‡ |
| 66-p-347 | AF318573*§ | AF31S573*§ | AF318573*§ | AF318573*§ | AF31S573*§ | AF318573*§ |
| Buf. | AY847330 | AY847308 | AY847338 | AY847324 | AY847344 | AY143157*‡ |
| 130 | AY857329 | AY847307 | AY847337 | AY847323 | AY847343 | AY143156*‡ |
| 108 | AY847328 | AY847306 | AY847336 | AY847322 | AY847342 | AY143155*‡ |
| M40 | AY847333 | AY847311 | AY847340 | AY847326 | AY847347 | AY143159*‡ |

### Supplementary Table S1. Primers used to amplify BoHV-4 genome sequences

The position of each primer is described according to the sequence of strain 66-p-347 (Zimmerman et al., 2001).

| Region | Nucleotide position | Type | Sequence (5'→3') |
|---|---|---|---|
| Intergenic region 71-73 and ORF 73 | 98866-95903 | Forward | GAGAGTGATTTGTAGTGT |
| | 98972-98993 | Forward | CAAAGAGATCTAGGTTGTCTGG |
| | 100123-100107 | Reverse | AGTTAGGAGACGTTCACCAACG |
| | 100337-100318 | Reverse | ATGCCGGGCCGCCATCAG |

### LEGEND TO FIGURES

Fig. 1. Flowchart of stages performed to produce BoHV-4 ORF73 recombinants using BAC cloning and prokaryotic recombination technologies.
Fig. 2. (a) Determination of ORF73 kinetic class of transcription. MDBK cells were mock-infected or infected with BoHV-4 V. test strain, in absence or presence of CHX or PAA. Twenty-four hours p.i., expression of IE Bo5, E ORF21, L ORF22, ORF71 and ORF73 was studied using an RT-PCR approach as described in Methods. mRNA and cDNA represent PCR products generated when the RT was omitted from the reactions and RT-PCR products, respectively. (b) Northern blot analysis was performed on total RNA harvested from mock-infected and infected MDBK cells 4, 8 and 24 h p.i. with the BoHV-4 V. test strain. ORF73 was used as probe. To optimize the visualization of the bands in the 24h p.i. sample, the corresponding line was exposed for a shorter period (right column). Weak and intense bands identified on the blot are marked with open arrows and arrows, respectively. (c) and (d) Characterisation of ORF73 transcripts by RT-PCR. Cytoplasmic RNA extracted 24 h p.i. was analysed by RT-PCR using the pairs of primers described in panel (c). For each pair of primers the size of the expected amplicon is indicated. The results of the PCR are presented in panel (d). PCR suggesting the polycistronic nature of ORF73 transcript are marked with asterisk.
Fig. 3. Structural analysis of BoHV-4 recombinant strains. The DNA of the various intermediates was analysed by *Hin*dIII restriction (left panel) and further tested by Southern blot (right panel) using probes corresponding to ORF73 and *gal*K cassette. Arrows and open arrows indicate restriction fragments containing the ORF73 and the *gal*K cassette, respectively. Markers sizes (MS) in kb are indicated on the left.
Fig. 4. Characterisation of BoHV-4 V. test strain and derived recombinant strains. (a) Epifluorescent analysis of viral plaques. MDBK cells were infected (MOI of 0.5 PFU/cell) with BoHV-4 V. test (i-iii), V. test BAC G (iv-vi), V. test BAC G excised (vii-ix), V. test BAC G ΔORF73 (x-xii), V. test BAC G ΔORF73 excised (xiii-xv), V. test BAC G ORF73 revertant (xvi-xviii) and V. test BAC G ORF73 revertant excised strains (xix-xxi). The cells were then overlaid with MEM containing 5% FCS and 0.6% carboxymethylcellulose to obtain isolated plaques. Three days p.i., plaques were revealed by indirect immunofluorescent staining using Mab 35 and Alexa Fluor 568-conjugated goat anti-mouse as primary and secondary antibodies, respectively. Each set of three horizontal panels represents analysis of the same plaques. EGFP and Alexa Fluor 568 fluorescences are shown in the first and the second column, respectively; while the merged EGFP and Alexa Fluor 568 signals are shown in the third column. The side of each panel corresponds to 75 µm of the specimen. (b) Replication kinetics of recombinant strains. Replication kinetics of V. test BAC G ΔORF73 excised strain (triangle) and V. test BAC G ORF73 revertant excised strain (cross) were compared with those of the parental V. test BAC G excised strain (square) as described in Methods. The data presented are the means +/- SD of triplicate measurements.
Fig. 5. BoHV-4 episome persistence and replication in BOMAC cells. (a) BOMAC cells were mock-infected (open circle) or infected with V. test BAC G (square), V. test BAC G ΔORF73 (triangle) or V. test BAC G ORF73 (cross). Episome persistence was then measured over time as described in Methods. Each value represents the mean +/- SD of triplicate measurements of the percentage of EGFP-positive cells based on the analysis of 10,000 cells. (b) 48 h p.i., BOMAC and MDBK cells (used as positive control for viral replication) were stained with Mab 35 raised against BoHV-4 gB and analysed by flow cytometry to investigate BoHV-4 replication. The results presented are based on the analysis of 10,000 cells.
Fig. 6. Rabbit infection with BoHV-4 ORF73 recombinants. Groups consisting of 3 rabbits were mock-infected or infected with V. test BAC G, V. test BAC G ΔORF73 and V. test BAC G ORF73 revertant excised strains, (a) Real-Time PCR relative quantification of BoHV-4 genome. DNA was extracted from the PBMC at the different times post-inoculation and from the spleen 64 days post-inoculation. Data are expressed as the number of BoHV-4 ORF8 gene copies per 10⁵ rabbit beta-globin gene copies for each rabbit, (b) Rabbit anti-BoHV-4 humoral response. Sera were collected at different times post-inoculation and the titre of anti-BoHV-4 antibodies was estimated by ELISA. Assays were made in triplicate for each rabbit. Each value represents the mean +/- SD of the data obtained for the three rabbits of each group.

### LITERATURE REFERENCES

Ballestas, M. E., Chatis, P. A. & Kaye, K. M. (1999). Efficient persistence of extrachromosomal KSHV DNA mediated by latency-associated nuclear antigen. Science 284, 641-4.
Barbera, A. J., Chodaparambil, J. V., Kelley-Clarke, B., Joukov, V., Walter, J. C., Luger, K. & Kaye, K. M. (2006). The nucleosomal surface as a docking station for Kaposi's sarcoma herpesvirus LANA. Science 311, 856-61.
Boudry, C., Markine-Goriaynoff, N., Delforge, C., Springael, J. Y., de Leval, L., Drion, P., Russell, G., Haig, D. M., Vanderplasschen, A. F. & Dewals, B. (2007). The A5 gene of alcelaphine herpesvirus 1 encodes a constitutively active G-protein-coupled receptor that is non-essential for the induction of malignant catarrhal fever in rabbits. J Gen Virol 88, 3224-33.
Cai, X., Lu, S., Zhang, Z., Gonzalez, C. M., Damania, B. & Cullen, B. R. (2005). Kaposi's sarcoma-associated herpesvirus expresses an array of viral microRNAs in latently infected cells. Proc Natl Acad Sci U S A 102, 5570-5.
Calderwood, M., White, R. E., Griffiths, R. A. & Whitehouse, A. (2005). Open reading frame 73 is required for herpesvirus saimiri A11-S4 episomal persistence. J Gen Virol 86, 2703-8.
Costes, B., Fournier, G., Michel, B., Delforge, C., Raj, V. S., Dewals, B., Gillet, L., Drion, P., Body, A., Schynts, F., Lieffrig, F. & Vanderplasschen, A. (2008). Cloning of the Koi Herpesvirus Genome as an Infectious Bacterial Artificial Chromosome Demonstrates That Disruption of the Thymidine Kinase Locus Induces Partial Attenuation in Cyprinus carpio koi. 10.1128/JVI.00211-08. J. Virol. 82, 4955-4964.
Dewals, B., Boudry, C., Farnir, F., Drion, P.-V. & Vanderplasschen, A. (2008). Malignant Catarrhal Fever Induced by Alcelaphine herpesvirus 1 is Associated with Proliferation of CD8+ T Cells Supporting a Latent Infection. PLoS ONE 3, e1627.
Dewals, B., Thirion, M., Markine-Goriaynoff, N., Gillet, L., de Fays, K., Minner, F., Daix, V., Sharp, P. M. & Vanderplasschen, A. (2006). Evolution of Bovine herpesvirus 4: recombination and transmission between African buffalo and cattle. J Gen Virol 87, 1509-19.
Donofrio, G., Cavirani, S., Vanderplasschen, A., Gillet, L. & Flammini, C. F. (2006). Recombinant bovine herpesvirus 4 (BoHV-4) expressing glycoprotein D of BoHV-1 is immunogenic and elicits serum-neutralizing antibodies against BoHV-1 in a rabbit model. Clin Vaccine Immunol 13, 1246-54.
Donofrio, G. & van Santen, V. L. (2001). A bovine macrophage cell line supports bovine herpesvirus-4 persistent infection. J Gen Virol 82, 1181-1185.
Ebrahimi, B., Dutia, B. M., Roberts, K. L., Garcia-Ramirez, J. J., Dickinson, P., Stewart, J. P., Ghazal, P., Roy, D. J. & Nash, A. A. (2003). Transcriptome profile of murine gammaherpesvirus-68 lytic infection. J Gen Virol 84, 99-109.
Fowler, P., Marques, S., Simas, J. P. & Efstathiou, S. (2003). ORF73 of murine herpesvirus-68 is critical for the establishment and maintenance of latency. J Gen Virol 84, 3405-16.
Friborg, J., Jr., Kong, W., Hottiger, M. O. & Nabel, G. J. (1999). p53 inhibition by the LANA protein of KSHV protects against cell death. Nature 402, 889-94.
Gillet, L., Daix, V., Donofrio, G., Wagner, M., Koszinowski, U. H., China, B., Ackermann, M., Markine-Goriaynoff, N. & Vanderplasschen, A. (2005). Development of bovine herpesvirus 4 as an expression vector using bacterial artificial chromosome cloning. J Gen Virol 86, 907-17.
Krithivas, A., Young, D. B., Liao, G., Greene, D. & Hayward, S. D. (2000). Human herpesvirus 8 LANA interacts with proteins of the mSin3 corepressor complex and negatively regulates Epstein-Barr virus gene expression in dually infected PEL cells. J Virol 74, 9637-45.
Lan, K., Kuppers, D. A., Verma, S. C. & Robertson, E. S. (2004). Kaposi's sarcoma-associated herpesvirus-encoded latency-associated nuclear antigen inhibits lytic replication by targeting Rta: a potential mechanism for virus-mediated control of latency. J Virol 78, 6585-94.
Lan, K., Kuppers, D. A., Verma, S. C., Sharma, N., Murakami, M. & Robertson, E. S. (2005). Induction of Kaposi's sarcoma-associated herpesvirus latency-associated nuclear antigen by the lytic transactivator RTA: a novel mechanism for establishment of latency. J Virol 79, 7453-65.
Li, H., Gailbreath, K., Flach, E. J., Taus, N. S., Cooley, J., Keller, J., Russell, G. C., Knowles, D. P., Haig, D. M., Oaks, J. L., Traul, D. L. & Crawford, T. B. (2005). A novel subgroup of rhadinoviruses in ruminants. J Gen Virol 86, 3021-3026.
Li, M., Lee, H., Yoon, D. W., Albrecht, J. C., Fleckenstein, B., Neipel, F. & Jung, J. U. (1997). Kaposi's sarcoma-associated herpesvirus encodes a functional cyclin. J Virol 71, 1984-91.
Lim, C., Gwack, Y., Hwang, S., Kim, S. & Choe, J. (2001). The transcriptional activity of cAMP response element-binding protein-binding protein is modulated by the latency associated nuclear antigen of Kaposi's sarcoma-associated herpesvirus. J Biol Chem 276, 31016-22.
Lu, F., Day, L., Gao, S. J. & Lieberman, P. M. (2006). Acetylation of the latency-associated nuclear antigen regulates repression of Kaposi's sarcoma-associated herpesvirus lytic transcription. J Virol 80, 5273-82.
Markine-Goriaynoff, N., Georgin, J. P., Goltz, M., Zimmermann, W., Broll, H., Wamwayi, H. M., Pastoret, P. P., Sharp, P. M. & Vanderplasschen, A. (2003). The core 2 beta-1,6-N-acetylglucosaminyltransferase-mucin encoded by bovine herpesvirus 4 was acquired from an ancestor of the African buffalo. J Virol 77, 1784-92.
Martinez-Guzman, D., Rickabaugh, T., Wu, T. T., Brown, H., Cole, S., Song, M. J., Tong, L. & Sun, R. (2003). Transcription program of murine gammaherpesvirus 68. J Virol 77, 10488-503.
Naeem, K., Caywood, D. D., Werdin, R. E. & Goyal, S. M. (1990). Evaluation of pregnant rabbits as a laboratory model for bovid herpesvirus-4 infection. Am J Vet Res 51, 640-4.
Osorio, F. A., Reed, D. E. & Rock, D. L. (1982). Experimental infection of rabbits with bovine herpesvirus-4: acute and persistent infection. Vet Microbiol 7, 503-13.
Radkov, S. A., Kellam, P. & Boshoff, C. (2000). The latent nuclear antigen of Kaposi sarcoma-associated herpesvirus targets the retinoblastoma-E2F pathway and with the oncogene Hras transforms primary rat cells. Nat Med 6, 1121-7.
Renne, R., Barry, C., Dittmer, D., Compitello, N., Brown, P. O. & Ganem, D. (2001). Modulation of cellular and viral gene expression by the latency-associated nuclear antigen of Kaposi's sarcoma-associated herpesvirus. J Virol 75, 458-68.
Roizman, B. & Pellett, P. E. (2007). The family Herpesviridae :a brief introduction. In Fields Virology (fifth edition), pp. 2479-2499. Edited by H. P. M. Knipe D.M. Philadelphia: Lippincott Williams & Wilkins, a Wolters Kluwer Business.
Sambrook, J. & Russell, D. W. (2001). Molecular Cloning: A Laboratory Manual. In Chapter 7 : Extraction, Purification and Analysis of mRNA from Eukaryotic Cells, Third Edition edn, pp. 7.1-7.45. Edited by N. Irwin. New-York: Cold Spring Harbor Lab.
Sarid, R., Flore, O., Bohenzky, R. A., Chang, Y. & Moore, P. S. (1998). Transcription mapping of the Kaposi's sarcoma-associated herpesvirus (human herpesvirus 8) genome in a body cavity-based lymphoma cell line (BC-1). J Virol 72, 1005-12.
Sarid, R., Wiezorek, J. S., Moore, P. S. & Chang, Y. (1999). Characterization and cell cycle regulation of the major Kaposi's sarcoma-associated herpesvirus (human herpesvirus 8) latent genes and their promoter. J Virol 73, 1438-46.
Stabel, J. R. & Stabel, T. J. (1995). Immortalization and characterization of bovine peritoneal macrophages transfected with SV40 plasmid DNA. Vet Immunol Immunopathol 45, 211-20.
Thiry, E., Bublot, M., Dubuisson, J., Van Bressem, M. F., Lequarre, A. S., Lomonte, P., Vanderplasschen, A. & Pastoret, P. P. (1992). Molecular biology of bovine herpesvirus type 4. Vet Microbiol 33, 79-92.
Thome, M., Schneider, P., Hofmann, K., Fickenscher, H., Meinl, E., Neipel, F., Mattmann, C., Burns, K., Bodmer, J. L., Schroter, M., Scaffidi, C., Krammer, P. H., Peter, M. E. & Tschopp, J. (1997). Viral FLICE-inhibitory proteins (FLIPs) prevent apoptosis induced by death receptors. Nature 386, 517-21.
Thompson, J., Gibson, T., Plewniak, F., Jeanmougin, F. & Higgins, D. (1997). The CLUSTAL _X windows interface: flexible strategies for multiple sequence alignment aided by quality analysis tools. 10.1093/nar/25.24.4876. Nucl. Acids Res. 25, 4876-4882.
Vanderplasschen, A., Bublot, M., Pastoret, P. P. & Thiry, E. (1993). Restriction maps of the DNA of cervid herpesvirus 1 and cervid herpesvirus 2, two viruses related to bovine herpesvirus 1. Arch Virol 128, 379-88.
Warming, S., Costantino, N., Court, D. L., Jenkins, N. A. & Copeland, N. G. (2005). Simple and highly efficient BAC recombineering using galK selection. Nucleic Acids Res 33, e36.
Weck, K. E., Kim, S. S., Virgin, H. I. & Speck, S. H. (1999). Macrophages are the major reservoir of latent murine gammaherpesvirus 68 in peritoneal cells. J Virol 73, 3273-83.
Ye, F. C., Zhou, F. C., Yoo, S. M., Xie, J. P., Browning, P. J. & Gao, S. J. (2004). Disruption of Kaposi's sarcoma-associated herpesvirus latent nuclear antigen leads to abortive episome persistence. J Virol 78, 11121-9.
Zimmermann, W., Broll, H., Ehlers, B., Buhk, H. J., Rosenthal, A. & Goltz, M. (2001). Genome sequence of bovine herpesvirus 4, a bovine Rhadinovirus, and identification of an origin of DNA replication. J Virol 75, 1186-94.

## Claims

1. Mutant Bovine Herpesvirus type 4 (BoHV-4) virus **characterised in that** it is not capable of making a functional ORF73 protein, whereby said BoHV-4 virus is **characterised in that** it has a mutation in the ORF73 gene; **in that** said virus comprises a second deletion; and **in that** said second deletion is a deletion in the ORF71-gene encoding a v-FLIP, the ORF16-gene encoding a v-Bcl-2 homologue, the Bo10 gene encoding glycoprotein gp80 or the ORF21-gene encoding thymidine kinase.

2. Mutant BoHV-4 virus according to claim 1 **characterised in that** said virus comprises a heterologous DNA sequence.

3. Mutant BoHV-4 virus according to claim 2, **characterised in that** said heterologous DNA sequence encodes an immunogenic protein of another virus or microorganism pathogenic to animals that are sensitive to BoHV-4 infection, preferably to ruminants, and/or a cytokine.

4. Mutant BoHV-4 virus according to claim 2 or 3, **characterised in that** said heterologous DNA sequence is inserted in the ORF73 gene.

5. Mutant BoHV-4 virus according to any of claims 1- 4 for use in a vaccine.

6. Vaccine for combating BoHV-4 infection, **characterised in that** said vaccine comprises a mutant BoHV-4 virus according to any of claims 1 - 4, and a pharmaceutically acceptable carrier.

7. Mutant BoHV-4 carrier vaccine, **characterised in that** said vaccine comprises a mutant BoHV-4 virus according to any of claims 2 - 4, and a pharmaceutically acceptable carrier.

8. Vaccine according to claim 6 or 7, **characterized in that** said vaccine is in a freeze-dried form.

9. A cell comprising a mutant BoHV-4 virus according to any of claims 1 - 4.

10. Method for the production of a mutant BoHV-4 virus according to any of claims 1 - 4, said method comprising the step of mutating the ORF73 gene so that it no longer encodes a functional ORF73 protein.

11. Diagnostic test kit for differentiating BoHV-4 field-virus infected animals from animals vaccinated with a mutant BoHV-4 vaccine according to claim 8 **characterized in that** said kit comprises PCR-primers against a fragment of ORF73 that is not present in mutant BoHV-4 virus according to any of claims 1 - 4.

## Patentansprüche

1. Mutantes Bovines Herpesvirus-Typ 4 (BoHV-4)-Virus, **dadurch gekennzeichnet, dass** es nicht in der Lage ist, ein funktionelles ORF73-Protein herzustellen, wobei das BoHV-4-Virus **dadurch gekennzeichnet ist, dass** es eine Mutation im ORF73-Gen aufweist; dass das genannte Virus eine zweite Deletion umfasst; und dass es sich bei der zweiten Deletion um eine Deletion im ORF71-Gen, das für ein v-FLIP kodiert, im ORF16-Gen, das für ein v-Bcl-2-Homolog kodiert, im Bo10-Gen, das für das Glykoprotein gp80 kodiert, oder im ORF21-Gen, das für Thymidinkinase kodiert, handelt.

2. Mutantes BoHV-4-Virus nach Anspruch 1, **dadurch gekennzeichnet, dass** das Virus eine heterologe DNA-Sequenz umfasst.

3. Mutantes BoHV-4-Virus nach Anspruch 2, **dadurch gekennzeichnet, dass** die heterologe DNA-Sequenz für ein immunogenes Protein eines anderen Virus oder Mikroorganismus, der pathogen bei gegenüber BoHV-4-Infektion anfälligen Tieren, vorzugsweise bei Wiederkäuern, ist, und/oder für ein Cytokin kodiert.

4. Mutantes BoHV-4-Virus nach Anspruch 2 oder 3, **dadurch gekennzeichnet, dass** die heterologe DNA-Sequenz in das ORF73-Gen insertiert ist.

5. Mutantes BoHV-4-Virus nach beliebigen der Ansprüche 1 - 4 zur Verwendung in einem Impfstoff.

6. Impfstoff zur Bekämpfung einer BoHV-4-Infektion, **dadurch gekennzeichnet, dass** der Impfstoff ein mutantes BoHV-4-Virus nach beliebigen der Ansprüche 1 - 4 und einen pharmazeutisch annehmbaren Träger umfasst.

7. Mutanter-BoHV-4-Träger-Impfstoff, **dadurch gekennzeichnet, dass** der Impfstoff ein mutantes BoHV-4-Virus nach beliebigen der Ansprüche 2 - 4 und einen pharmazeutisch annehmbaren Träger umfasst.

8. Impfstoff nach Anspruch 6 oder 7, **dadurch gekennzeichnet, dass** der Impfstoff in gefriergetrockneter Form vorliegt.

9. Zelle, umfassend ein mutantes BoHV-4-Virus nach beliebigen der Ansprüche 1 - 4.

10. Verfahren zur Herstellung eines mutanten BoHV-4-Virus nach beliebigen der Ansprüche 1 - 4, wobei das Verfahren den Schritt des Mutierens des ORF73-Gens umfasst, so dass es nicht länger für ein funktionelles ORF73-Protein kodiert.

11. Diagnostisches Testkit zur Unterscheidung von mit BoHV-4-Feldvirus infizierten Tieren von mit einem mutanten BoHV-4-Impfstoff nach Anspruch 8 geimpften Tieren, **dadurch gekennzeichnet, dass** das Kit PCR-Primer gegen ein Fragment von ORF73 umfasst, das in mutantem BoHV-4 Virus nach beliebigen der Ansprüche 1 - 4 nicht vorhanden ist.

## Revendications

1. Virus de l'herpès bovin de type 4 (BoHV-4) mutant **caractérisé en ce qu'**il n'est pas capable de fabriquer une protéine ORF73 fonctionnelle, ledit virus BoHV-4 étant **caractérisé en ce qu'**il comporte une mutation dans le gène ORF73 ; **en ce que** ledit virus comprend une deuxième délétion ; et **en ce que** ladite deuxième délétion est une délétion dans le gène ORF71 codant pour un v-FLIP, le gène ORF16 codant pour un homologue de v-Bcl-2, le gène Bo10 codant pour la glycoprotéine gp80 ou le gène ORF21 codant pour la thymidine kinase.

2. Virus BoHV-4 mutant selon la revendication 1, **caractérisé en ce que** ledit virus comprend une séquence d'ADN hétérologue.

3. Virus BoHV-4 mutant selon la revendication 2, **caractérisé en ce que** ladite séquence d'ADN hétérologue code pour une protéine immunogène d'un autre virus ou micro-organisme pathogène pour des animaux qui sont sensibles à une infection par BoHV-4, de préférence des ruminants, et/ou une cytokine.

4. Virus BoHV-4 mutant selon la revendication 2 ou 3, **caractérisé en ce que** ladite séquence d'ADN hétérologue est insérée dans le gène ORF73.

5. Virus BoHV-4 mutant selon l'une quelconque des revendications 1 à 4 pour utilisation dans un vaccin.

6. Vaccin pour lutter contre une infection par BoHV-4, **caractérisé en ce que** ledit vaccin comprend un virus BoHV-4 mutant selon l'une quelconque des revendications 1 à 4, et un véhicule pharmaceutiquement acceptable.

7. Vaccin à porteur de BoHV-4 mutant, **caractérisé en ce que** ledit vaccin comprend un virus BoHV-4 mutant selon l'une quelconque des revendications 2 à 4, et un véhicule pharmaceutiquement acceptable.

8. Vaccin selon la revendication 6 ou 7, **caractérisé en ce que** ledit vaccin est sous une forme lyophilisée.

9. Cellule comprenant un virus BoHV-4 mutant selon l'une quelconque des revendications 1 à 4.

10. Procédé de production d'un virus BoHV-4 mutant selon l'une quelconque des revendications 1 à 4, ledit procédé comprenant l'étape de mutation du gène ORF73 de sorte qu'il ne code plus pour une protéine ORF73 fonctionnelle.

11. Trousse de test de diagnostic pour différencier des animaux infectés par le virus de terrain BoHV-4 des animaux vaccinés avec un vaccin à BoHV-4 mutant selon la revendication 8, **caractérisée en ce que** ladite trousse comprend des amorces de PCR contre un fragment d'ORF73 qui n'est pas présent dans le virus BoHV-4 mutant selon l'une quelconque des revendications 1 à 4.
